# EUROPEAN PATENT APPLICATION

(11) **EP 2 594 194 A1**
(43) Date of publication of application: **22.05.2013**
(21) Application number: 12158524.4
(22) Date of filing: 08.03.2012
(51) Int. Cl.: A61B 5/022, A61B 5/00

(54) **Blood pressure measurement system**

(30) Priority: 17.11.2011 TW 100142156
(71) Applicant: Zoetronics Technology Co., Ltd., Taipei City 104 (TW)
(72) Inventor: Chen, Jen-Ran, 116 Taipei City (TW); Lee, Ren-Guey, 106 Taipei City (TW); Lai, Chien-Chih, 515 Changhua County (TW); Wang, Hsi-Wen, 235 New Taipei City (TW); Chen, Chun-Chang, 404 Taichung City (TW)
(74) Representative: Becker Kurig Straus

(57) **Abstract**

A blood pressure measurement system (200) includes an article of clothing (202), an occluding cuff (204), a blood pressure measurement unit (206), a first transmission module (210), and an operation module (212). The clothing (202) includes tarpaulin (2022). The occluding cuff (204) is disposed at an inner side of the tarpaulin (2022). The blood pressure measurement unit (206) is disposed at the inner side of the tarpaulin (2022) for measuring blood pressure of a user to generate a blood pressure measurement result of the user, controlling a removable inflation module (2082) to inflate the occluding cuff (204), controlling a deflate valve (2042) to deflate the occluding cuff (204), and transmitting the blood pressure measurement result. The first transmission module (210) is used for receiving the blood pressure measurement result. The operation module (212) is coupled to the first transmission module (210) for calculating diastolic blood pressure, systolic blood pressure, and/or pulses of the user according to the blood pressure measurement result.

## Description

### Field of the Invention

The present invention relates to a blood pressure measurement system according to the pre-characterizing clause of claim 1.

### Background of the Invention

A blood pressure measurement result is a significant criterion of hypertension and cardiovascular diseases. If the blood pressure of a user can be measured over a long-term at fixed time intervals to generate a blood pressure measurement result of the user, the blood pressure measurement result of the user can help a doctor to effectively diagnose changes of cardiovascular diseases of the user. Because the sphygmomanometer needs an inflatable motor and the user needs a display to watch a blood pressure measurement result easily when the sphygmomanometer measures blood pressure of the user, the sphygmomanometer usually has a larger volume, which makes it inconvenient to carry, resulting in a goal of long-term blood pressure measurement being implemented with difficulty.

### Summary of the Invention

This in mind, the present invention aims at providing a blood pressure measurement system that not only can reduce the volume of a blood pressure measurement unit, but can also record a blood pressure measurement result of a user for a long-term to a cloud platform of the Internet.

This is achieved by a blood pressure measurement system according to claim 1. The dependent claims pertain to corresponding further developments and improvements.

As will be seen more clearly from the detailed description following below, the claimed blood pressure measurement system includes an article of clothing, an occluding cuff, a blood pressure measurement unit, a first transmission module, and an operation module. The clothing includes tarpaulin, where the tarpaulin is sewn into the clothing. The occluding cuff is disposed at an inner side of the tarpaulin. The blood pressure measurement unit is also disposed at the inner side of the tarpaulin for measuring the blood pressure of a user to generate a blood pressure measurement result of the user, receiving an inflation signal to control a removable inflation module to inflate the occluding cuff, receiving a deflation signal to control a deflate valve of the occluding cuff to deflate the occluding cuff, and transmitting the blood pressure measurement result of the user. The first transmission module receives the blood pressure measurement result of the user. The operation module is coupled to the first transmission module for calculating diastolic blood pressure, systolic blood pressure, and/or pulses of the user according to the blood pressure measurement result of the user.

### Brief Description of the Drawings

In the following, the invention is further illustrated by way of example, taking reference to the accompanying drawings. Thereof:
FIG. 1 is a diagram illustrating an electronic sphygmomanometer utilizing a resonance method to record the blood pressure of the user,
FIG. 2 is a diagram illustrating a blood pressure measurement system according to an embodiment, and
FIG. 3 is a diagram illustrating a blood pressure measurement system according to another embodiment.

### Detailed Description

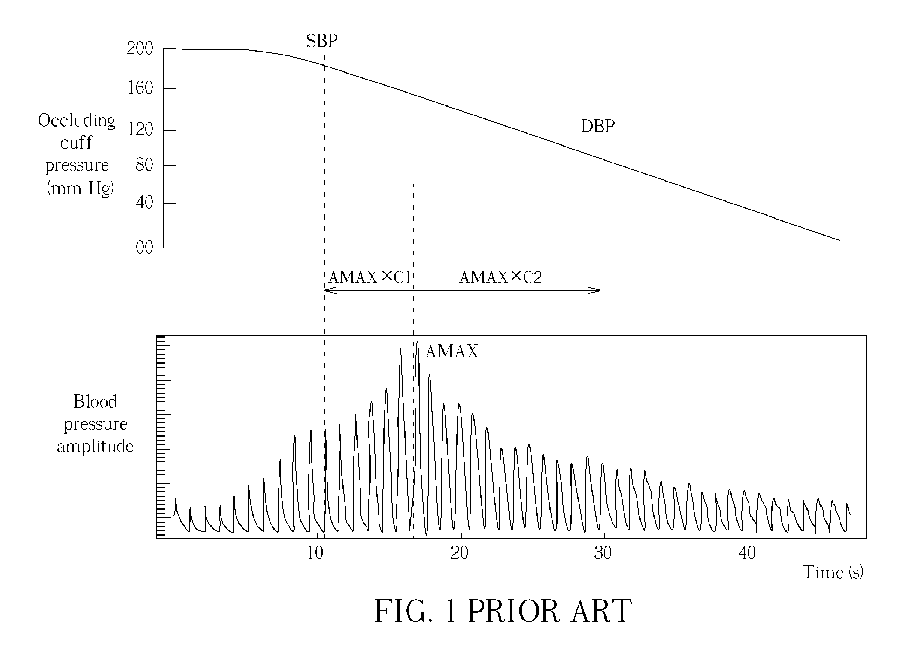

Please refer to FIG. 1. FIG. 1 is a diagram illustrating an electronic sphygmomanometer utilizing a resonance method to record the blood pressure of the user. At first, the sphygmomanometer inflates an occluding cuff surrounding an arm of the user to block blood of the arm of the user. Then, a deflate valve of the occluding cuff deflates the occluding cuff. As shown in FIG. 1, during the occluding cuff going from inflation to deflation, a manometer coupled to the occluding cuff can detect maximum blood pressure AMAX of the user, and calculate diastolic blood pressure DBP, systolic blood pressure SBP, and pulses of the user according to the maximum blood pressure AMAX, and constants C1 and C2.

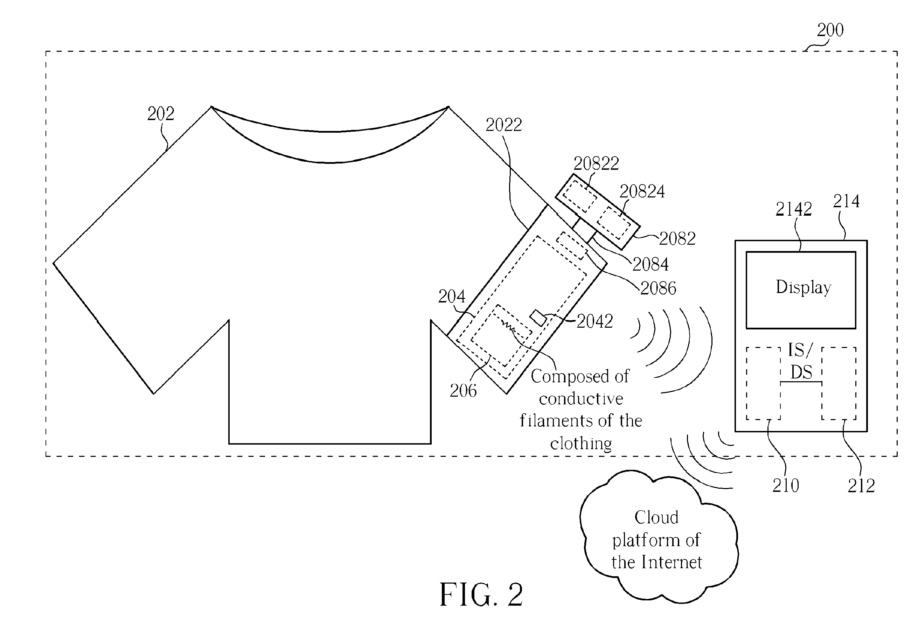

Please refer to FIG. 2. FIG. 2 is a diagram illustrating a blood pressure measurement system 200 according to an embodiment of the present invention. The blood pressure measurement system 200 includes an article of clothing 202, an occluding cuff 204, a blood pressure measurement unit 206, a first transmission module 210, and an operation module 212. As shown in FIG. 2, the clothing 202 includes tarpaulin 2022, where the clothing 202 is short-sleeved clothing, and the tarpaulin 2022 is sewn into the clothing 202 and located at a cuff of the clothing 202. But, in another embodiment of the present invention, the clothing 202 is long-sleeved clothing, and the tarpaulin 2022 is located at a cuff of the long-sleeved clothing, or the tarpaulin 2022 is at an upper arm location of the long-sleeved clothing. The occluding cuff 204 is disposed at an inner side of the tarpaulin 2022. The blood pressure measurement unit 206 is also disposed at the inner side of the tarpaulin 2022 for measuring blood pressure of a user to generate a blood pressure measurement result of the user, where the blood pressure measurement unit 206 can be a highly integrated system-on-a-chip (SOC), or a miniaturized sphygmomanometer. In addition, at least one passive component of the blood pressure measurement unit 206 is composed of conductive filaments of the clothing 202, where the at least one passive component can be a resistor, an inductor, or a capacitor. The blood pressure measurement unit 206 is further used for receiving an inflation signal IS to control a removable inflation module 2082 to inflate the occluding cuff 204, receiving a deflation signal DS to control a deflate valve 2042 of the occluding cuff 204 to deflate the occluding cuff 204, and transmitting the blood pressure measurement result of the user. As shown in FIG. 2, the removable inflation module 2082 is connected to the tarpaulin 2022 through a metal tenon 2084 of the tarpaulin 2022, and the removable inflation module 2082 inflates the occluding cuff 204 through a one-way water valve 2086 of the tarpaulin 2022. But, the present invention is not limited to the removable inflation module 2082 being connected to the tarpaulin 2022 through the metal tenon 2084 of the tarpaulin 2022. That is to say, the removable inflation module 2082 can be connected to the tarpaulin 2022 through at least one metal tenon, at least one metal button, at least one conductive velcro, or at least one magnet of the tarpaulin. In addition, the removable inflation module 2082 includes a battery 20822 which is used for driving an inflatable motor 20824 of the removable inflation module 2082. Therefore, the removable inflation module 2082 can utilize the inflatable motor 20824 to inflate the occluding cuff 204 through the one-way water valve 2086 of the tarpaulin 2022. As shown in FIG. 2, the first transmission module 210 communicates with the blood pressure measurement unit 206 through a wireless transmission manner. That is to say, the first transmission module 210 receives the blood pressure measurement result of the user from the blood pressure measurement unit 206 through the wireless transmission manner, where the wireless transmission manner can be a wireless Local Area Network (WLAN), a Zigbee (IEEE 802.15.4), a Bluetooth, a Bluetooth Low Energy (BLE), a Worldwide Interoperability for Microwave Access (WiMAX), a Global System for Mobile Communications (GSM), a General Packet Radio Service (GPRS), a Third Generation (3G), or an Actor Network Theory+ (Ant+) technology. But, in another embodiment of the present invention, the first transmission module 210 communicates with the blood pressure measurement unit 206 through a transmission line. The operation module 212 is coupled to the first transmission module 210 for calculating diastolic blood pressure, systolic blood pressure, and/or pulses of the user according to the blood pressure measurement result of the user. The first transmission module 210 and the operation module 212 are located outside the clothing 202 and included in a cell phone 214. Therefore, the operation module 212 can utilize a display 2142 of the cell phone 214 to display the diastolic blood pressure, the systolic blood pressure, and/or the pulses of the user. But, the present invention is not limited to the first transmission module 210 and the operation module 212 being included in the cell phone 214. That is to say, the first transmission module 210 and the operation module 212 can also be included in a smart phone, a tablet computer, a notebook computer, a personal digital assistant or a desktop computer. In addition, the inflation signal IS and the deflation signal DS are generated by the operation module 212, and the inflation signal IS and the deflation signal DS are transmitted to the blood pressure measurement unit 206 through the first transmission module 210. As shown in FIG. 2, the first transmission module 210 further uploads the diastolic blood pressure, the systolic blood pressure, and/or the pulses of the user to a cloud platform (such as a medical service cloud platform) of the Internet through the wireless transmission manner. Then, the cloud platform can perform long-term recording, analysis, and/or monitoring of blood variation of the user. But, in another embodiment of the present invention, the first transmission module 210 uploads the diastolic blood pressure, the systolic blood pressure, and/or the pulses of the user to the cloud platform of the Internet through a cable.

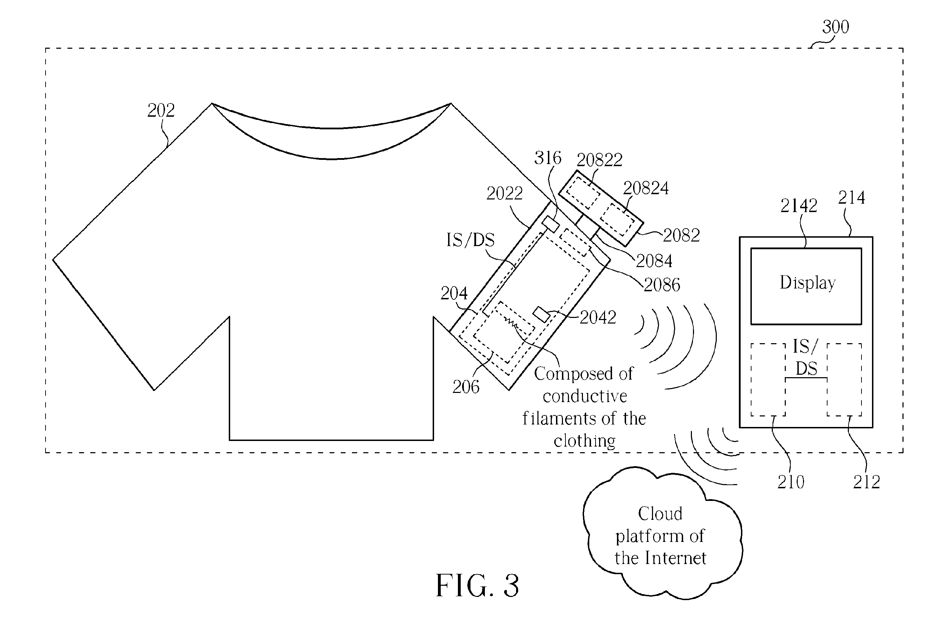

Please refer to FIG. 3. FIG. 3 is a diagram illustrating a blood pressure measurement system 300 according to another embodiment. A difference between the blood pressure measurement system 300 and the blood pressure measurement system 200 is that the tarpaulin 2022 of the blood pressure measurement system 300 further includes a key 316, where the key 316 is coupled to the blood pressure measurement unit 206. As shown in FIG. 3, the user can utilize the operation module 212 of the cell phone 214 to generate an inflation signal IS and a deflation signal DS, then the operation module 212 transmits the inflation signal IS and the deflation signal DS to the blood pressure measurement unit 206 through the wireless transmission manner/the wired transmission manner. Or, the user can utilize the key 316 to generate an inflation signal IS and a deflation signal DS to the blood pressure measurement unit 206. Further, subsequent operational principles of the blood pressure measurement system 300 are the same as those of the blood pressure measurement system 200, so further description thereof is omitted for simplicity.

To sum up, when the user wears the clothing with the blood pressure measurement system provided by the present invention, the blood pressure measurement system utilizes the blood pressure measurement unit disposed at the inner side of the tarpaulin of the clothing to measure blood pressure of the user, and transmits a blood pressure measurement result of the user to the operation module. Then, the operation module can calculate diastolic blood pressure, systolic blood pressure, and/or pulses of the user according to the blood pressure measurement result of the user, and upload the diastolic blood pressure, the systolic blood pressure, and/or the pulses of the user to the cloud platform of the Internet through the first transmission module. In addition, the blood pressure measurement unit can be a highly integrated system-on-a-chip and at least one passive component of the blood pressure measurement unit is composed of conductive filaments of the clothing. Therefore, the present invention not only can reduce volume of the blood pressure measurement unit, but can also record the blood pressure measurement result of the user long-term to the cloud platform of the Internet. Then, the cloud platform can perform long-term recording, analysis, and/or monitoring of blood variation of the user.

## Claims

1. A blood pressure measurement system, comprising:
an article of clothing (202) comprising tarpaulin (2022) sewn into the clothing (202);
an occluding cuff (204) disposed at an inner side of the tarpaulin (2022);
a first transmission module (210) for receiving a blood pressure measurement result of a user;
an operation module (212) coupled to the first transmission module (210) for calculating diastolic blood pressure, a systolic blood pressure, and/or pulses of the user according to the blood pressure measurement result of the user ; and
**characterized by**:
a blood pressure measurement unit (206) disposed at the inner side of the tarpaulin (2022) for measuring blood pressure of the user to generate the blood pressure measurement result of the user, receiving an inflation signal to control a removable inflation module (2082) to inflate the occluding cuff (204), receiving a deflation signal to control a deflate valve (2042) of the occluding cuff (204) to deflate the occluding cuff (204), and transmitting the blood pressure measurement result of the user.

2. The blood pressure measurement system of claim 1, **characterized in that** at least one passive component of the blood pressure measurement unit (206) is composed of conductive filaments of the clothing (202).

3. The blood pressure measurement system of claim 2, **characterized in that** the at least one passive component is a resistor, an inductor, or a capacitor.

4. The blood pressure measurement system of claim 1, **characterized in that** the blood pressure measurement unit (206) is a system-on-a-chip or a miniaturized sphygmomanometer.

5. The blood pressure measurement system of claim 1, **characterized in that** the first transmission module (210) and the operation module (212) are located outside the clothing (202), and the first transmission module (210) further uploads the diastolic blood pressure, the systolic blood pressure, and/or the pulses of the user to a cloud platform via the Internet.

6. The blood pressure measurement system of claim 1, **characterized in that** the first transmission module (210) and the operation module (212) are comprised by a cell phone (214), a smart phone, a tablet computer, a notebook computer, a personal digital assistant or a desktop computer.

7. The blood pressure measurement system of claim 1, **characterized in that** the clothing (202) is long-sleeved clothing, and the tarpaulin (2022) is located at a cuff of the clothing (202).

8. The blood pressure measurement system of claim 1, **characterized in that** the clothing (202) is long-sleeved clothing, and the tarpaulin (2022) is at an upper arm location of the long-sleeved clothing.

9. The blood pressure measurement system of claim 1, **characterized in that** the clothing (202) is short-sleeved clothing, and the tarpaulin (2022) is located at a cuff of the clothing (202).

10. The blood pressure measurement system of claim 1, **characterized in that** the removable inflation module (2082) comprises a battery (20822).

11. The blood pressure measurement system of claim 1, **characterized in that** the removable inflation module (2082) is connected to the tarpaulin (2022) through at least one metal tenon (2084), at least one metal button, at least one conductive velcro, or at least one magnet, and the removable inflation module (2082) inflates the occluding cuff (204) through a one-way water valve (2086) of the tarpaulin (2022).

12. The blood pressure measurement system of claim 1, **characterized in that** the inflation signal and the deflation signal are generated by the operation module (212), and the inflation signal and the deflation signal are transmitted to the blood pressure measurement unit (206) through the first transmission module (210).

13. The blood pressure measurement system of claim 1, **characterized in that** the inflation signal and the deflation signal are generated by a key (316) of the tarpaulin (2022), and the key (316) is coupled to the blood pressure measurement unit (206).

14. The blood pressure measurement system of claim 1, **characterized in that** the blood pressure measurement unit (206) communicates with the first transmission module (210) through a Wireless Local Area Network, a Zigbee, a Bluetooth, a Bluetooth Low Energy, a Worldwide Interoperability for Microwave Access, a Global System for Mobile Communications, a General Packet Radio Service, a Third Generation, or an Actor Network Theory+ technology.

15. The blood pressure measurement system of claim 1, **characterized in that** the blood pressure measurement unit (206) communicates with the first transmission module (210) through a transmission line.
